# EUROPÄISCHE PATENTANMELDUNG

(11) **EP 2 213 228 A2**
(43) Veröffentlichungstag der Anmeldung: **04.08.2010**
(21) Anmeldenummer: 09180069.8
(22) Anmeldetag: 21.12.2009
(51) Int. Cl.: A61B 5/06, A61F 2/06, A61B 17/00, A61B 19/00

(54) **Degradations- und Integritätsmessgerät für absorbierbare Metallimplantate**

(30) Priorität: 30.01.2009 DE 102009000501
(71) Anmelder: Biotronik VI Patent AG, 6341 Baar (CH)
(72) Erfinder: Klocke, Björn, 8006, Zürich (CH); Skerl, Olaf, 18209, Bad Doberan (DE)
(74) Vertreter: Lindner-Vogt, Karin L.

(57) **Zusammenfassung**

Beschrieben wird ein Messgerät, umfassend einen Sensorkatheter (12,22,32,42), eine Auswerteeinheit (13) und in dem Sensorkatheter (12,22,32,42) angeordnete und mit der Auswerteeinheit (13) verbundene Mittel (15,24,35,45) zur Aufnahme eines Magnetfeldes (1) und Wandlung des Magnetfeldes (1) in ein elektrisches Messsignal, wobei die Auswerteeinheit (13) ausgebildet ist, das elektrische Messsignal auszuwerten. Ziel ist eine Degradations- und Integritätsmessung für absorbierbare Metallimplantate.

## Beschreibung

### Hintergrund der Erfindung

Für medizinische Zwecke werden absorbierbare metallische Implantate wie Stents, Klammern, orthopädische Implantate und viele andere mehr verwendet bzw. befinden sich in der Entwicklung. Die Implantate werden dem Patienten eingesetzt und sollen über eine bestimmte Zeit eine gewünschte Funktion erfüllen, schließlich jedoch ohne eine weitere Operation zu deren Entfernung wieder aus dem Körper gelangen. Die Implantate werden daher aus speziellen absorbierbaren Legierungen wie Magnesium-, Eisen- oder Zinklegierungen gefertigt, wodurch der Körper des Patienten die Implantate über die Zeit abbauen kann, so dass sie über den normalen Stoffwechsel schlussendlich ausgeschieden werden können.

Es ist allerdings schwierig, die Geschwindigkeit des Absorptionsprozesses mit hinreichender Genauigkeit einzuschätzen, da diese von Patient zu Patient variieren kann. Für die klinische Praxis ist es aber wichtig, den Degradationszustand und den mechanischen Zustand (inkl. der Integrität) der Implantate zu kennen. Bekannte Verfahren wie Röntgen, Kernspin, Ultraschall, intravaskulärer Ultraschall (IVUS) oder optische Kohärenztomographie (OCT) eignen sich hierzu nur eingeschränkt, weil sie die Umwandlungsprozesse im Metall, die sich teilweise im und unterhalb des mikroskopischen Maßstabs abspielen, mangels räumlicher Auflösung und qualitativer chemischer Trennschärfe nicht ausreichend abzubilden vermögen. Es besteht daher ein Bedarf an einer Vorrichtung, die eine Messung des Degradationszustandes von absorbierbaren metallischen Implantaten und dadurch des Anteils des absorbierten Materials und der mechanische Integrität der Implantate erlaubt.

Offenbarung der Erfindung

Die Erfindung führt ein Messgerät ein, welches einen Sensorkatheter, eine Auswerteeinheit und in dem Sensorkatheter angeordnete und mit der Auswerteeinheit verbundene Mittel zur Aufnahme eines Magnetfeldes und Wandlung des Magnetfeldes in ein elektrisches Messsignal umfasst. Die Auswerteeinheit ist ausgebildet, das elektrische Messsignal auszuwerten.

In dieser einfachsten Ausführung des Messgeräts wird das Magnetfeld außerhalb des Körpers des Patienten und damit getrennt vom Messgerät beispielsweise durch eine Erregerspule erzeugt. Dabei wird das als Katheter ausgeführte Messgerät genutzt, um das Magnetfeld intrakorporal in der Nähe des Implantats zu messen.

Die Erfindung erlaubt eine zuverlässige Detektion eines absorbierbaren metallischen Implantates und die Bestimmung der Menge des noch nicht absorbierten metallischen Materials. Bei kleinen, tief im Gewebe implantierten Implantaten ermöglicht der Sensorkatheter einen minimal-invasiven Eingriff, allerdings ist auch eine nicht-invasive Verwendung des Messgerätes denkbar, wenn das Implantat nahe an der Hautoberfläche gelegen und genügend groß für eine messtechnische Erfassung ist.

Besonders bevorzugt enthält das Messgerät jedoch selbst Mittel zur Erzeugung des Magnetfeldes, beispielsweise einen Wechselstromgenerator und eine in dem Sensorkatheter angeordnete und mit dem Wechselstromgenerator verbundene Erregerspule zur Erzeugung eines Magnetfeldes. Der Wechselstromgenerator ist dabei ausgebildet, einen Wechselstrom zu erzeugen und an die Erregerspule zur Erzeugung des Magnetfeldes abzugeben.

Die im Sensorkatheter platzierte Erregerspule kann mit dem Sensorkatheter in den Körper eingeführt werden und erzeugt dort ein magnetisches Wechselfeld. Das magnetische Wechselfeld generiert wiederum in einem in der Nähe befindlichen metallischen Körper Wirbelströme, welche ihrerseits magnetische Felder erzeugen, die dem magnetischen Wechselfeld der Erregerspule entgegenwirken. Das magnetische Wechselfeld der Erregerspule wird daher umso schwächer, je mehr Metall im Umfeld der Erregerspule vorhanden ist, womit der gewünschte Rückschluss auf die Menge und den Ort des verbleibenden metallischen Materials möglich wird. Die Erfindung lässt sich natürlich auch zur Lokalisierung und ggf. dem Wiederfinden von nicht absorbierbaren metallischen Implantaten verwenden. In diesem Fall ist eine quantitative Information über die Menge des metallischen Materials unerheblich. Auch ist es möglich, Risse in einem metallischen Implantat aufzuspüren, weil die Wirbelströme aufgrund der nichtleitfähigen Unterbrechung im Metallkörper abgeschwächt werden und das Magnetfeld der Erregerspule weniger schwächen, als bei intaktem Metallkörper zu erwarten gewesen wäre. Dadurch wird es möglich, eine zuverlässige Integritätsprüfung von metallischen Implantaten durchzuführen.

Bei einer bevorzugten Ausführung der Erfindung ist der Wechselstromgenerator mit der Auswerteeinheit verbunden und ausgebildet, den Wechselstrom mit einer von der Auswerteeinheit in einem Frequenzbereich zwischen einer unteren Grenzfrequenz und einer oberen Grenzfrequenz vorgebbaren Frequenz zu erzeugen.

Diese Ausführungsform erlaubt die Erzeugung von magnetischen Wechselfeldern mindestens einer vorgebbaren Frequenz. Dies trägt der Tatsache Rechnung, dass Körperflüssigkeiten wie beispielsweise Blut aufgrund der darin gelösten Ionen ebenfalls leitfähig sein können, so dass auch hier Wirbelströme mit den oben beschriebenen Wirkweisen induziert werden. Um nun jedoch eine Schwächung des Magnetfeldes der Erregerspule durch ein metallisches Implantat von einer solchen durch beispielsweise Blut unterscheiden zu können, ist es hilfreich, die Messung bei mindestens einer wählbaren Frequenz durchführen zu können. So können die erwähnten Ionen im Blut bei einer hohen Frequenz aufgrund ihrer Masse und ihrer eingeschränkten Beweglichkeit nicht mehr oder nicht mehr vollständig folgen, während dies für die im Metall frei beweglichen Elektronen immer noch möglich ist. Es wird somit möglich, die Messung unter Berücksichtigung des direkten Umfeldes des Sensorkatheters durchzuführen.

Vorzugsweise beträgt die untere Grenzfrequenz zwischen 5 kHz und 20 kHz. Die obere Grenzfrequenz beträgt bevorzugt zwischen 1 MHz und 10 MHz. Z.B. ist der Wechselstromgenerator ausgebildet, Frequenzen im Bereich von 5 kHz bis hin zu 10 MHz zu erzeugen. Es sind auch Anregungen denkbar, die durch nicht-sinusförmige Anregungen (z.B. ein Rechtecksignal bzw. ein Burstsignal) mehrere Grundfrequenzen im relevanten Frequenzbereich abdecken. Als eine weitere Alternative ist es denkbar, dass das Gerät die Frequenz automatisch variiert und die Messfrequenz auf die konkrete Messsituation anpasst.

Es ist denkbar, dass eine Messfrequenz so gewählt wird, dass die Wirbelströme in Resonanz oder nahe der Resonanz mit elektrischen bzw. geometrischen Eigenschaften des Implantates sind. Mit anderen Worten kann die Messfrequenz abhängig von der Eigenfrequenz der vom Implantat vorgegebenen Schwingkreise (implizite kapazitive und induktive Elemente) gemacht werden. Damit ist es auch denkbar, Informationen sowohl zum gesamten Massenverlust des Implantates einerseits und zur Trennung von elektrischen Verbindungen (z.B. Struts bei degradablen Stents) bzw. Integrität andererseits zu gewinnen und in getrennten Messwerten zu erfassen.

Bei einer besonders bevorzugten Ausführungsvariante ist die Auswerteeinheit ausgebildet, dem Wechselstromgenerator zu einem ersten Messzeitpunkt eine erste zu erzeugende Frequenz und zu einem zweiten Messzeitpunkt eine zweite, von der ersten verschiedene zu erzeugende Frequenz vorzugeben und das elektrische Messsignal unter Berücksichtigung der ersten und der zweiten Frequenz auszuwerten. Werden zwei oder mehr Messungen bei jeweils verschiedenen Frequenzen vorgenommen, kann der oben beschriebene Einfluss von störenden Elementen wie Blut in der Auswertung reduziert oder sogar eliminiert werden. Vorzugsweise ist die zweite Frequenz wenigstens fünf- bis zehnmal so groß wie die erste Frequenz.

Die Auswerteeinheit ist bei einer Variante der Erfindung ausgebildet, einen Phasenwinkel zwischen dem eingespeisten Wechselstrom und dem elektrischen Messsignal zu bestimmen. Die bereits erwähnte Abschwächung des Magnetfeldes wirkt sich auf die effektive Impedanz der Anordnung aus, welche den Phasenwinkel zwischen dem Wechselstrom und dem elektrischen Messsignal entsprechend beeinflusst. Dementsprechend kann die Auswertung des elektrischen Messsignals in der Auswerteeinheit über die messtechnische Bestimmung des Phasenwinkels zwischen dem Wechselstrom und dem elektrischen Messsignal erfolgen.

Alternativ oder zusätzlich kann die Auswerteeinheit ausgebildet sein, den Betrag des elektrischen Messsignals zu bestimmen. Da sich die Anwesenheit eines metallischen Körpers - wie bereits beschrieben - auf die Stärke des resultierenden Magnetfeldes auswirkt, kann die Auswertung des Messsignals auch auf der Bestimmung dessen Betrages beruhen.

Bei einer besonders bevorzugten Ausführung der Erfindung können die Mittel zur Aufnahme des Magnetfeldes und Wandlung des Magnetfeldes in ein elektrisches Messsignal die Erregerspule selbst sein. In diesem Fall wird die Auswertung aufgrund des Vergleichs von Wechselstrom und der Spannung über die Spule vorgenommen, wobei die Spannung über der Spule das elektrische Messsignal darstellt.

Alternativ können die Mittel zur Aufnahme des Magnetfeldes und Wandlung des Magnetfeldes in ein elektrisches Messsignal eine Empfängerspule sein. In diesem Fall beinhaltet der Sensorkatheter ein Spulenpaar oder eine Doppelspule. Die Auswertung beruht auf der durch das Magnetfeld in der Empfängerspule induzierten Spannung, welche in diesem Fall das elektrische Messsignal darstellt.

In einer weiteren alternativen Ausführungsform können die Mittel zur Aufnahme des Magnetfeldes und Wandlung des Magnetfeldes in ein elektrisches Messsignal ein Magnetfeldsensor, insbesondere ein Hall-Sensor oder GMR-Sensor, sein.

### Kurzbeschreibung der Abbildungen

Die Erfindung wird im Folgenden anhand von Abbildungen von Ausführungsbeispielen näher erläutert. Es zeigen
- Fig. 1: ein Blockdiagramm eines erfindungsgemäßen Messgerätes;
- Fig. 2: einen Querschnitt durch die Spitze einer ersten Ausführungsform eines Sensorkatheters;
- Fig. 3: einen Querschnitt durch die Spitze einer zweiten Ausführungsform eines Sensorkatheters; und
- Fig. 4: einen Querschnitt durch die Spitze einer dritten Ausführungsform eines Sensorkatheters.

### Ausführliche Beschreibung der Abbildungen

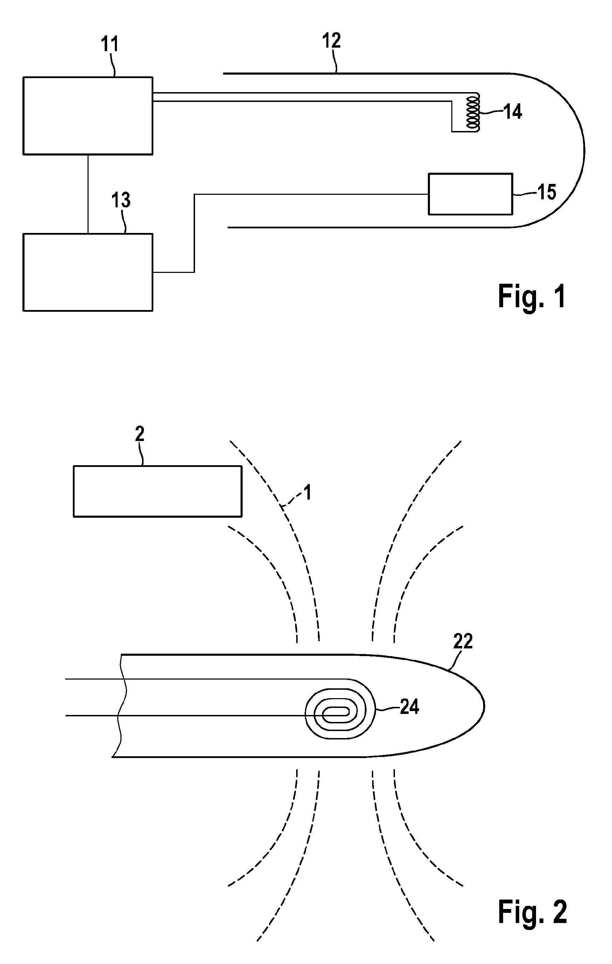

Fig. 1 zeigt ein Blockdiagramm eines erfindungsgemäßen Messgerätes. In einem Sensorkatheter 12 sind eine Erregerspule 14 für die Erzeugung eines Magnetfeldes und Mittel 15 zur Aufnahme des Magnetfeldes und Wandlung des Magnetfeldes in ein elektrisches Messsignal angeordnet. Die Erregerspule 14 ist mit einem Wechselstromgenerator 11 verbunden, der ausgebildet ist, einen Wechselstrom zu erzeugen und an die Erregerspule 14 abzugeben. Im abgebildeten Beispiel ist der Wechselstromgenerator 11 mit einer Auswerteeinheit 13 verbunden, welche ihrerseits mit den Mitteln 15 zur Aufnahme des Magnetfeldes und Wandlung des Magnetfeldes in ein elektrisches Messsignal verbunden ist. Die Auswerteeinheit 13 erhält von den Mitteln 15 zur Aufnahme des Magnetfeldes und Wandlung des Magnetfeldes in das elektrische Messsignal das elektrische Messsignal und wertet dieses aus. Die Auswerteeinheit 13 des Beispiels ist außerdem dazu ausgebildet, dem Wechselstromgenerator 11 eine Frequenz des vom Wechselstromgenerator 11 zu erzeugenden Wechselstroms vorzugeben. Die Auswertung des elektrischen Messsignals kann nach Methoden, die bei Metalldetektoren zum Suchen von vergrabenen Metallgegenständen (Orts- und Mengenbestimmung des metallischen Materials) oder bei der Rissprüfung von Metallrohren (Integritätsprüfung aufgrund der Abschwächung von Wirbelströmen durch nichtleitende Risse) verwendet werden, vorgenommen werden.

Fig. 2 zeigt einen Querschnitt durch die Spitze einer ersten Ausführungsform eines Sensorkatheters 22. In dem Sensorkatheter 22 ist eine Erregerspule 24 platziert, welche ausgebildet ist, ein Magnetfeld 1 zu erzeugen. Das Magnetfeld 1 ist, wie auch bei den folgenden Abbildungen, aus Gründen der besseren Darstellung gestrichelt im Bildhintergrund dargestellt, durchdringt jedoch auch den Sensorkatheter 22. Die Erregerspule 24 kann als mikrosystemtechnisch gefertigte Planarspule ausgeführt sein und fungiert im abgebildeten Beispiel gleichzeitig als Mittel zur Aufnahme des Magnetfeldes und Wandlung des Magnetfeldes in das elektrische Messsignal. Wird in die Erregerspule 24 ein Wechselstrom geeigneter Frequenz eingespeist, verändert sich deren komplexe Impedanz Z in Abhängigkeit vom Vorhandensein eines gut leitfähigen, beispielsweise metallischen, Körpers 2. Die im leitfähigen Körper 2 durch das magnetische Wechselfeld induzierten Wirbelströme erzeugen ihrerseits ein magnetisches Wechselfeld, das dem magnetischen Wechselfeld 1 der Erregerspule 24 entgegenwirkt, was sich in der Impedanz Z der Erregerspule 24 widerspiegelt. Die in der Abbildung nicht gezeigte Auswerteeinheit enthält Mittel zur Messung der komplexen Impedanz Z der Erregerspule 24 und zur Auswertung sowie ggf. zur Darstellung oder Aufzeichnung der Messwerte. Die Impedanzmessung erfolgt vorzugsweise bei zwei oder mehr unterschiedlichen Frequenzen. Die Impedanzmessungen werden hinsichtlich Betrag und Phase der komplexen Impedanz mittels bekannter geeigneter Verfahren ausgewertet. Die Größe und die Anzahl des oder der gut leitfähigen Körper 2, beispielsweise die Restmenge eines resorbierbaren metallischen Stents in einem Blutgefäß, können auf diese Weise bestimmt werden.

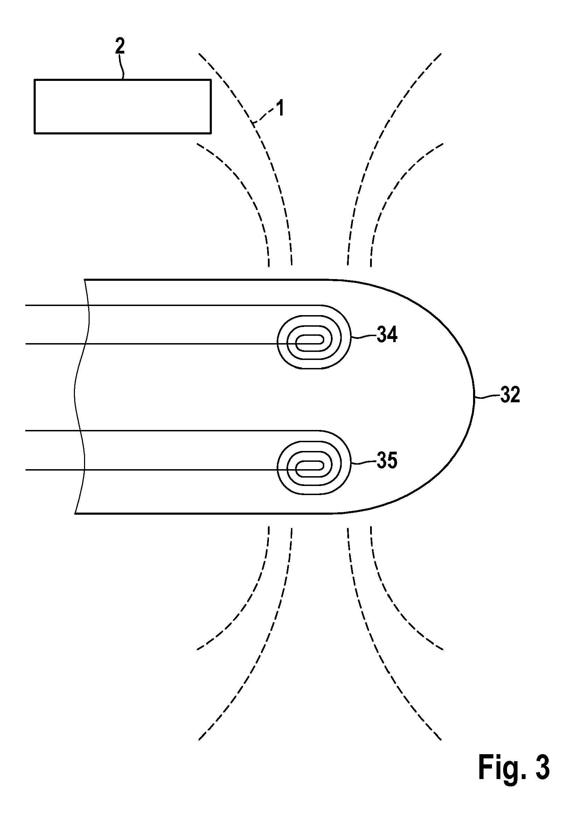

Fig. 3 zeigt einen Querschnitt durch die Spitze einer zweiten Ausführungsform eines Sensorkatheters 32. Die Anordnung entspricht im Wesentlichen der der ersten Ausführungsform von Fig. 2, verfügt aber im Unterschied zu jener über ein aus einer Erregerspule 34 und einer Empfängerspule 35 gebildetes Spulenpaar. Das von der Erregerspule 34 erzeugte Magnetfeld 1 erzeugt durch Induktion in der Empfängerspule 35 eine entsprechende elektrische Spannung als elektrisches Messsignal. Die Empfängerspule erfüllt in dieser Ausführungsform somit die Funktion der Mittel zur Aufnahme des Magnetfeldes und Wandlung des Magnetfeldes in das elektrische Messsignal. Das Magnetfeld 1 wird aufgrund der schon beschriebenen Wirkungen eines im Magnetfeld 1 befindlichen metallischen Körpers 2 abgeschwächt, was sich entsprechend in der Größe der in der Empfängerspule 35 induzierten Spannung niederschlägt.

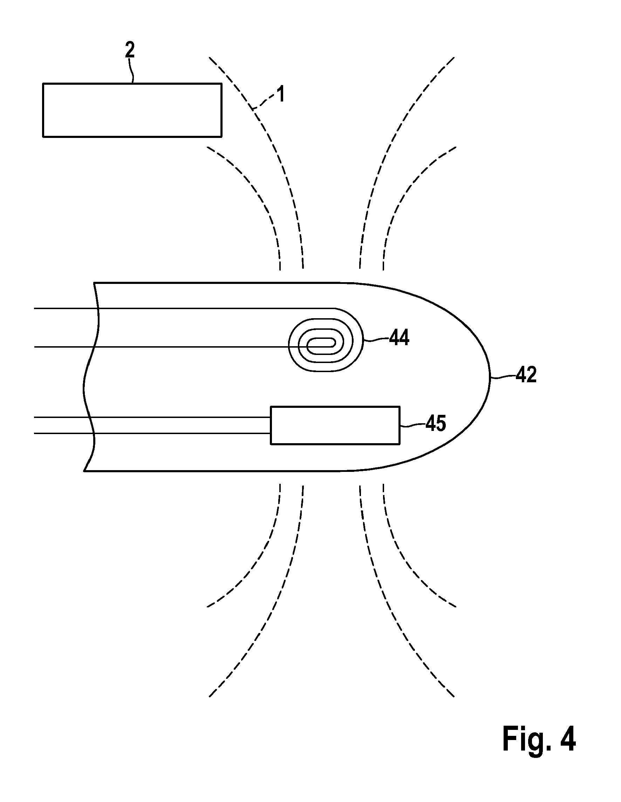

Fig. 4 zeigt einen Querschnitt durch die Spitze einer dritten Ausführungsform eines Sensorkatheters 42. Die Anordnung entspricht wiederum im Wesentlichen der der in den Fig. 2 und 3 gezeigten Ausführungsformen, verfügt aber im Unterschied zu jenen über einen Magnetfeldsensor 45, welcher hier die Funktion der Mittel zur Aufnahme des Magnetfeldes und Wandlung des Magnetfeldes in das elektrische Messsignal übernimmt. Der Magnetfeldsensor 45 kann beispielsweise als Hall-Sensor oder als GMR-Sensor (Giant Magneto Resistance) ausgeführt sein. Erregerspule 44 und Magnetfeldsensor 45 können gemeinsam in einer Einheit mit mikrosystemtechnischen Mitteln realisiert werden.

## Patentansprüche

1. Messgerät, umfassend einen Sensorkatheter (12, 22, 32, 42), eine Auswerteeinheit (13) und in dem Sensorkatheter (12, 22, 32, 42) angeordnete und mit der Auswerteeinheit (13) verbundene Mittel (15, 24, 35, 45) zur Aufnahme eines Magnetfeldes (1) und Wandlung des Magnetfeldes (1) in ein elektrisches Messsignal, wobei die Auswerteeinheit (13) ausgebildet ist, das elektrische Messsignal auszuwerten.

2. Das Messgerät von Anspruch 1, außerdem umfassend einen Wechselstromgenerator (11) und eine in dem Sensorkatheter (12, 22, 32, 42) angeordnete und mit dem Wechselstromgenerator (11) verbundene Erregerspule (14, 24, 34, 44) zur Erzeugung eines Magnetfeldes (1), wobei der Wechselstromgenerator (11) ausgebildet ist, einen Wechselstrom zu erzeugen und an die Erregerspule (14, 24, 34, 44) zur Erzeugung des Magnetfeldes (1) abzugeben.

3. Das Messgerät von Anspruch 2, bei dem der Wechselstromgenerator (11) mit der Auswerteeinheit (13) verbunden und ausgebildet ist, den Wechselstrom mit einer von der Auswerteeinheit (13) in einem Frequenzbereich zwischen einer unteren Grenzfrequenz und einer oberen Grenzfrequenz vorgebbaren Frequenz zu erzeugen.

4. Das Messgerät von Anspruch 3, bei dem die untere Grenzfrequenz zwischen 5 kHz und 20 kHz beträgt.

5. Das Messgerät von einem der Ansprüche 3 oder 4, bei dem die obere Grenzfrequenz zwischen 1 MHz und 10 MHz beträgt.

6. Das Messgerät von einem der Ansprüche 3 bis 5, bei dem die Auswerteeinheit (13) ausgebildet ist, dem Wechselstromgenerator (11) zu einem ersten Messzeitpunkt eine erste zu erzeugende Frequenz und zu einem zweiten Messzeitpunkt eine zweite, von der ersten verschiedene zu erzeugende Frequenz vorzugeben und das elektrische Messsignal unter Berücksichtigung der ersten und der zweiten Frequenz auszuwerten.

7. Das Messgerät von einem der Ansprüche 2 bis 6, bei dem die Auswerteeinheit (13) ausgebildet ist, einen Phasenwinkel zwischen dem Wechselstrom und dem elektrischen Messsignal zu bestimmen.

8. Das Messgerät von einem der vorhergehenden Ansprüche, bei dem die Auswerteeinheit (13) ausgebildet ist, den Betrag des elektrischen Messsignals zu bestimmen.

9. Das Messgerät nach einem der Ansprüche 2 bis 8, bei dem die Mittel (15, 24, 35, 45) zur Aufnahme des Magnetfeldes (1) und Wandlung des Magnetfeldes (1) in ein elektrisches Messsignal die Erregerspule (14, 24, 34, 44) sind.

10. Das Messgerät von einem der Ansprüche 1 bis 8, bei dem die Mittel (15, 24, 35, 45) zur Aufnahme des Magnetfeldes (1) und Wandlung des Magnetfeldes (1) in ein elektrisches Messsignal eine Empfängerspule (35) sind.

11. Das Messgerät von einem der Ansprüche 1 bis 8, bei dem die Mittel (15, 24, 35, 45) zur Aufnahme des Magnetfeldes (1) und Wandlung des Magnetfeldes (1) in ein elektrisches Messsignal ein Magnetfeldsensor (45), insbesondere ein Hall-Sensor oder GMR-Sensor, sind.

12. Verwendung eines Messgeräts nach einem der vorhergehenden Ansprüche zur Bestimmung des Degradationszustandes von Implantaten mit degradablen metallischen Anteilen.
